# EUROPEAN PATENT SPECIFICATION

(11) **EP 0 431 135 B1**
(45) Date of publication and mention of the grant of the patent: **26.03.2003**
(21) Application number: 90910074.5
(22) Date of filing: 21.06.1990
(51) Int. Cl.: C12N 15/87, C12N 5/10, C12N 15/00

(54) **PARTICLE-MEDIATED TRANSFORMATION OF ANIMAL SOMATIC CELLS**
TRANSFORMATION VON TIERISCHEN SOMATISCHEN ZELLEN MITTELS PARTIKELN
TRANSFORMATION AU MOYEN DE PARTICULES DE CELLULES SOMATIQUES ANIMALES

(30) Priority: 26.06.1989 US 371869; 14.03.1990 US 494933
(43) Date of publication of application: 12.06.1991
(73) Proprietor: Powderject Vaccines, Inc., Madison, Wisconsin 53711 (US)
(72) Inventor: BRILL, Winston, J., Madison, WI 53711 (US); McCABE, Dennis, E., Middleton, WI 53562 (US); YANG, Ning-Sun, Verona, WI 53593 (US)
(74) Representative: Woods, Geoffrey Corlett
(86) International application number: US9003522
(87) International publication number: WO91000359

(56) References cited:
- EP-A- 0 301 749
- Particulate Science and Technology vol. 5, 1987, WASHINGTON US pages 27 - 37; SANFORD,J.C. et al.: "Delivery of substances into cells and tissues using a particle bombardment process" see figures 1, 2
- BIOTECHNOLOGY vol. 6, May 1988, NEW YORK US pages 559 - 563; KLEIN, T.M. et al.: "Factors influencing gene delivery into zea mays cells by high-velocity_microprojectiles." see page 561, column 2 - page 562; figures
- SCIENCE. vol. 240, 1988, LANCASTER, PA US pages 1538 - 1541; JOHNSTON, S.A. et al.: "Mitochondrial transformation in yeast by bombardment with microprojectiles" see the whole document
- FEBS LETT vol. 244, no. 1, 01 February 1989, pages 65 - 67

## Description

### Field of the Invention

The present invention relates to the technologies of genetic transformation in general and relates, in particular, to strategies for the genetic transformation of the non-germ line cells of animals.

### Background of the Invention

Techniques have been developed for the genetic engineering of animals by which exogenous or foreign DNA can be inserted into the genomic DNA of animals.
Typically in the prior art such genetic transformation of animals is performed by microinjection or by the use of retroviral based transformation vectors the effect of which is to genetically transform an animal embryo at a relatively early stage in development. The foreign DNA is incorporated into the genome of the animal embryo and then becomes incorporated into the genome of each of the daughter cells which arise from that embryo. Such genetic transformations insert the inserted DNA into all of the cells and the resulting whole organism including the germ line or sex cells of the organism. This insures that the genetic trait is passed to the progeny of the transformed animal in a normal Mendellian fashion.

There are occasions in which it would be desirable to transform animal cells in situ so that the animal can be imbued with the gene product of a genetic construction without affecting the genetic makeup of the germ line of the animal. In particular, for human applications the use of such somatic cell transformation avoids many of the ethical and philosophical problems which would arise from human intervention with the germ lines of human beings. The genetically engineered somatic cells offers the ability to make genetic corrections for inherited genetic disorders which consist of inactive or deleted enzymes necessary for normal biological functioning. It is also possible that such genetic transformations of somatic cells, and not germ line cells, may be desirable for certain therapeutic applications. For example, certain proteins offering therapeutic utility to patients must be currently injected into patients on a periodic strict time-line basis. However, the periodic injection of large quantities of proteins, even if done frequently, can result in an over supply of the protein shortly after an injection and a diminished supply shortly before the next injection resulting in potentially toxic shock following the injection and an insufficient supply for therapeutic efficacy just prior to the subsequent injection. An alternative strategy might be to engineer the gene for the desired protein into somatic cells of the animal or human so that the transformed cells would produce the therapeutic protein at a consistent level while they are live. By introducing the transforming gene into somatic cells which have a pre-defined and ascertainable life expectancy, such as skin cells for example, it is possible to create such an in vivo therapeutic production system which is time limited in the administration of the protein dosage to the animal or person being treated. In veterinary applications it may be desirable to introduce hormones or other growth factors or proteins for animal improvement, therapeutic, or disease inhibiting purposes into somatic cell portions of the animal which are not transient but which stay with the animal for its life expectancy.

While the vast majority of efforts directed at transformation of animal organisms or animal cells in culture have been directed toward the use of microinjection techniques or retroviral transformation vectors, the apparatus used for the transformation technique in acordance with the present invention is based on a quite different methodology of transforming the foreign DNA into the genome of the transformed somatic cells. There is one suggestion in the prior art of an appartus containing some of the features which allow the apparatus used in the present invention to be particularly adapted for its present use. As disclosed by Klein *et al*, Nature, 327: 70-73 (1987), an instrument for the acceleration of very small particles of metal carrying DNA thereon has been demonstrated to be effective for the transformation of plant cells in culture. The transforming DNA is coated onto very small particles which are physically accelerated by actually being shot on a ballistic projectile into the tissues to be transformed. While this apparatus has been demonstrated to have utility in transforming plant cells in culture, it suffers from a deficiency in that the adjustability of the force of impact of its particles is lacking making it a difficult apparatus to use for transformation of organisms over a wide range of kinetic energies of insertion of the particles into the transformed tissue.

### Summary of the Invention

The present invention is concerned with the transformation *in vivo* of somatic cells in the skin of living animals using exogenous DNA, coding for the protein desired to be expressed in the somatic animal cells, that is coated onto small microparticles which are sufficiently small in size so as to be able to enter the cells of animals without disrupting their biological function.

Accordingly, the present invention provides carrier particles for use in a method of therapy by genetic transformation *in vivo* of somatic cells in the skin of a living animal, wherein the particles are of dense material, are small relative to the size of the said cells, and are coated with copies of an exogenous genetic construction that includes a protein-coding DNA sequence and flanking regulatory sequences effective to express the protein in the said cells.

A portion of the skin cells are genetically transformed. A number of cells in the skin of the animal are thus transformed *in vivo* to produce the protein coded by the exogenous gene. The carrier particles can be gold particles, for example gold particles 1-3µm in size. The animal is typically a human being.

### Brief Description of the Drawings

Fig. 1 is an exploded perspective view of apparatus used to perform the present invention.
Fig. 2 is a top plan view of the discharge chamber of the apparatus of Fig. 1.

### Description of the Preferred Embodiment

The present invention is directed toward the transformation of the somatic cells in the skin of living animals, including human beings. By somatic cells as used herein is meant those cells of an animal or human being which when transformed do not change the genetic character or makeup of any of the germ or sex cells of the organism, so that when the animal or human reproduces through normal biological forms of reproduction, the introduced exogenous genetic material is not passed to the biological progeny of the organism.

An exogenous, often chimeric, genetic construction is introduced into the somatic skin cells. Such exogenous genetic constructions consist of DNA from another organism, whether of the same or different species, which is introduced into somatic cells in the skin of a living animal through human manipulation, by the artificial introduction of genes into the somatic skin cells of the animal. The exogenous DNA construction includes a coding sequence for a protein of interest, together with flanking regulatory sequences effective to cause the expression of the protein coded for by the coding sequence in the transformed cells Examples of flunking regulatory sequences are a promoter sequence sufficient to initiate transcription and a terminator sequence sufficient to terminate the gene product, coded for by the gene, whether by termination of transcription or translation. Suitable transcriptional enhancers or enhancers of translational deficiency can be included in the exogenous gene construct to further assist the efficiency of the overall transformation process and expression of the protein result in the transformed cells. Other gene products than proteins may also be expressed by the inserted genetic construction. For example, the inserted construction could express a negative RNA strand effective either to suppress the expression of a native gene or to inhibit a disease pathology. The inserted construction could itself be RNA, as an alterative to DNA, if only transient expression of the gene product was desired.

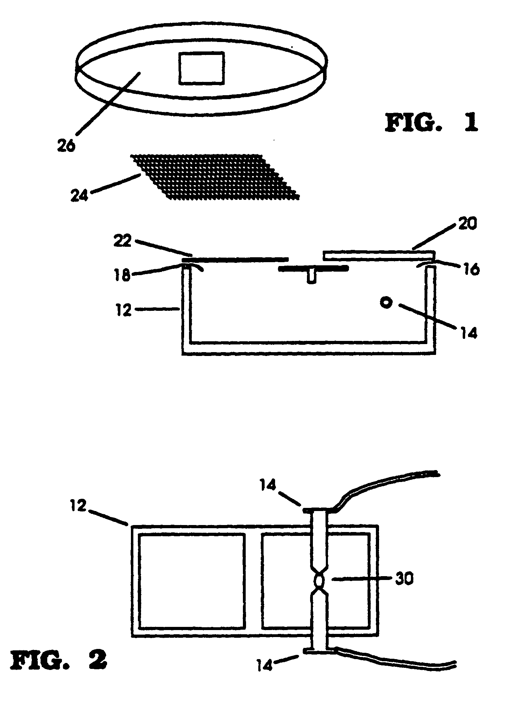

The present invention makes particular use of an apparatus for using an adjustable electric discharge to physically accelerate DNA coated onto small particles into the genetic material of somatic animal cells. A suitable apparatus for use within the present invention is illustrated in Fig. 1. The apparatus consists of a spark discharge chamber 12 into which are inserted two electrodes 14 which are spaced apart by a distance of approximately 1 - 2 mm. The spark discharge chamber is a horizontally extended rectangle having two openings 16 and 18 out its upward end. One opening 18 is covered by an access plate 20. The other opening, located opposite from the electrodes 14 is intended to be covered by a carrier sheet 22. The electrodes 14 are connected to a suitable adjustable source of electric discharge voltage. Such a source of electric discharge voltage would preferably include suitable electric switching connected to a capacitor of the 1 to 2 micro farad size range, with the amount of the voltage of the charge introduced into the capacitor being adjustable, such as through the use of an autotransformer, through a range of, for example, 1 to 50,000 volts. Suitable switching is provided so that the capacitor can be discharged through the electrodes 14 safely and conveniently by a user.

The carrier sheet 22 intended to be placed upon the opening 18 on the spark discharge chamber 12 is preferably a sheet of aluminized saran coated mylar. Above the opening in the discharge chamber, placed approximately 5 - 10 millimeters above it, is a retaining screen 24. Placed approximately 5 - 25 millimeters above the retaining screen is a target surface 26. In its use, the exogenous foreign gene construct intended to be transformed into the animal somatic cells is prepared by suitable DNA preparation techniques well known to one of ordinary skill in the art and it is dried onto small particles of a durable dense material such as gold, the particles typically being 1 to 3 microns in size. The carrier particles with the DNA dried thereon is then placed upon the carrier sheet 22 which is inserted on top of the spark discharge chamber 12. A target tissue, such as a live and anesthetized animal, is then placed adjacent to the target surface 26. Then a small droplet of water, approximately 2 - 4 microliters in size, is placed bridging between the ends of the electrodes 14. The access plate cover 20 is then placed over the top of the discharge chamber 12. At this point, the atmosphere between the carrier sheet 22 and the target is largely replaced with helium, by enclosing the apparatus and target and introducing helium in the enclosure in sufficient quantity to largely displace the atmospheric gases.

At this point the initiation of the spark discharge between the electrodes may be initiated by means of the use of the appropriate electronic switching. The force of the electric discharge bridges the spark discharge cap between the electrodes 14 instantly vaporizing the small droplet of water placed therebetween. The force of the vaporization of that water creates a shock wave within the spark discharge chamber 12 which radiates outward in all directions. The impact of the shock wave upon the carrier sheet 22 propels the carrier sheet 22 upwards with great velocity. The upwardly traveling carrier sheet 22 accelerates upward in direction until contacting the retaining screen 24. The presence of the helium provides less drag on the flight of the carrier sheet as well as less force for the shock wave to propagate to the target. At the retaining screen 24, the carrier sheet 22 is retained, and the DNA-coated particles previously applied thereto fly off of the carrier sheet and travel freely on toward the target surface. The particles therefor proceed into the target surface and enter the cells thereof. The momentum of the particles as they impact the surface of the target organism or tissue is adjustable based on the voltage of the initial electric discharge applied to the electrodes 14. Thus by variations in the amount of the electric energy discharged through the electrodes 14, the velocity by which the particles impact the target can be adjusted, and thus the depth of penetration of the particles into the tissue of a target, can be continuously adjusted over the range of adjustment of the electric discharge throughout the electrodes 14.

The apparatus of Fig. 1 has been previously demonstrated to be useful for the transformation of differentiated or undifferentiated tissue in a variety of forms including cellular masses in culture and whole growing organisms. It has been found through the work discussed herein that the apparatus is equally suitable for the transformation of somatic cells in the skin of a living animal. It is possible to transform portions of the skin of whole animals by anesthetizing the animal as appropriate for the species and type of animal and then placing the anesthetized animal over a hole cut in a planar surface which will act as the target surface. The portion of the animal exposed through the hole in the target surface 26 will therefore be the treated target tissue transformed by the transformation process.

### Examples

### a) Vectors used

The following examples make use of a pair of chimeric expression vectors constructed so as to express in animals the enzyme chloramphenicol acetyltransferase, which confers resistance to the antibiotic chloramphenicol. Both chimeric gene expression plasmids have been previously described and demonstrated to be effective in animal transfection studies. The plasmid pSV2cat was described by Gorman et al., Mol. Cell Biol., 2:1044-1051 (1982) and the expression vector pRSVcat was described by Walker et al., Nature, 306:557-561 (1983). The plasmid pSV2cat is a chimeric cat gene construction including the Simian virus 40 (SV40) early promoter, the chloramphenicol acetyltransferase coding region from the plasmid pBR322-Tn9, the SV40 t-antigen intron, and the SV40 early polyadenylation region carried in the pBR322 vector. The plasmid does not contain a complete SV40 viral genome and is not infectious. The plasmid pRSVcat is also a pBR322 base plasmid that includes a chimeric Rous Sarcoma virus (RSV) long terminal repeat and promoter fragment, the cat coding region from Tn9, an intron from the mouse beta-globulin gene and the polyadenylation region from the SV40 early transcription unit. This plasmid does also not contain a viral genome and is not infectious. does not contain a viral genome and is not infectious.

### b) Mammalian Somatic Cells In Vivo

Mice were anesthetized with chloroform. On each mouse, an area of approximately 1 cm² on its side was shaved. The mouse was then placed on a petri dish having a window cut in it with the shaved patch over the window.

DNA of pRSVcat was then coated onto 1-3 micron gold particles at a rate of 0.1 microgram of DNA per milligram of gold. The DNA was applied to the gold by precipitation with 25mM spermidine with 6% polyethylene glycol (m.w. 3,000) with the addition of CaCl₂.to a final concentration of 0.6 M. The DNA coated gold beads were then rinsed in a 100% ethanol and applied to the carrier sheet as an ethanolic suspension at a concentration of dried gold coated beads of 0.05 mg/cm² of the carrier sheet.

The petri dish with the mouse was placed over the apparatus of Figs. 1 and 2 as the target surface. Prior to the electric spark discharge, the area between the carrier sheet and the target was flushed with helium (4 liters/min) for 15 seconds to reduce atmospheric drag on the carrier sheet and any possible shock wave damage to the animal.

After the transformation event, the animals all appeared unharmed and they seemed to recover completely. No bruising or bleeding of any kind was observed. After 24 hours the mice were sacrificed and the skin patch was removed and assayed for cat activity. The assay was performed by testing for acetylation activity with a radio-labeled of C¹⁴. Radioactive decay of the acetylated product could then be used as a measure of transformed enzyme activity.

For the various electric discharge levels and controls used, the results are summarized in the following table.

| Conditions | Counts per 50 microliter | Total Protein Microgram/ul | Counts per 50 Microgram Protein |
|---|---|---|---|
| 12 KV voltage & 1 micron | 16,686 | 4.4 | 3792 |
| 16 KV voltage & 1 micron | 6,281 | 5.6 | 1121 |
| 12 KV voltage & 1 micron | 15,937 | 5.6 | 2854 |
| 12 KV voltage & 1 micron | 14,969 | 3.5 | 4276 |
| DNA + Kaolin (DNA soak control) | 123 | 4.3 | 28 |
| DNA + DMSO (DNA soak control) | 117 | 2.3 | 50 |
| No DNA (control) | 119 | 5.6 | 21 |

These results indicate cat activity of at least 100 times background levels. Thus a foreign gene was delivered and expressed in somatic cells without evidence of harm or damage to the animal.

### c) Amphibian Somatic Cells In Vivo

A (Xenopus) frog was anesthetized by chilling to 4° C. The chilled frog was also placed over a window cut in a petri dish lid and placed in the transformation apparatus of Figs. 1 and 2 in the same fashion as with the mice.

The conditions and procedure used for the mice were repeated for the frog except for the following. The DNA used was pSV2cat. The DNA coated gold beads were loaded onto the carrier sheet at a density of 0.1 mg/cm².

Again after the transformation process, the animal appeared entirely unharmed. Again no bruising or bleeding of the animal was detected. After 24 hours, the frog was sacrificed and the 1 cm² patch of skin transformed was removed and assayed for cat activity. The results are tabulated on the following table.

| Conditions | Counts per 50 microliter | Total Protein Microgram/ul | Counts per 50 microgram Protein |
|---|---|---|---|
| 12 KV (belly) | 13,149 | 2.1 | 6261 |
| 16 KV (back) | 17,570 | 4.0 | 4392 |
| Control (belly) | 153 | 1.4 | 109 |
| Control (back) | 145 | 4.1 | 32 |

Thus, in this example levels of cat activity were observed at least in excess of 50 times background. Thus delivery and expression of a foreign gene was achieved in somatic cells without any identifiable damage or injury to the animal.

### d) Amphibian Somatic Cells In Vivo - Systemic Product

In a second experiment on Xenopus, one animal was transformed under similar conditions, as above, but twice on the same frog (16 KV on its back, 12 KV on its belly). In this case only 0.05 mg/cm² instead of 0.1 mg/cm² DNA coated beads were used. The frog was sacrificed after 20 hours, and the transformed skin patches sampled. In addition, a portion of non-transformed skin (shielded at the time of blasting) was sampled for CAT activity. The results are summarized in the following table.

| Results | Counts/50ul | Total Protein mg/ul | Counts Per 50 ul Protein |
|---|---|---|---|
| 12 KV (belly) | 2,085 | 7.5 | 278 |
| 16 KV (back) | 9,343 | 8.6 | 1,086 |
| Untreated skin from elsewhere on the same toad | 1,301 | 5.1 | 255 |

Total activity in the transformed skin patches was reduced due to the lower bead loading rate, but the non-transformed skin sample clearly shows at least a 2 fold elevation above a non-transformed animal's skin, as in the previous experiment, thus showing a systemic accumulation of the enzyme produced in the transformed skin patches.

## Claims

1. Carrier particles for use in a method of therapy by genetic transformation *in vivo* of somatic cells in the skin of a living animal, wherein the particles are of dense material, are small relative to the size of the said cells, and are coated with copies of an exogenous genetic construction that includes a protein-coding DNA sequence and flanking regulatory sequences effective to express the protein in the said cells.

2. Particles according to claim 1, which are of gold.

3. Particles according to claim 2, which are 1-3 µm in size.

4. Particles according to any preceding claim, wherein the living animal is a human being.

## Patentansprüche

1. Trägerteilchen zur Verwendung bei einem Verfahren der Therapie durch genetische Transformation *in vivo* von somatischen Zellen in der Haut eines lebenden Tieres, wobei die Teilchen aus dichtem Material sind, im Vergleich zu der Größe der Zellen klein sind und mit Kopien einer exogenen genetischen Konstruktion, die eine Protein-codierende DNA-Sequenz und flankierende Regulationssequenzen, welche zur Expression des Proteins in den Zellen wirksam sind, einschließt, beschichtet sind.

2. Teilchen gemäß Anspruch 1, welche aus Gold sind.

3. Teilchen gemäß Anspruch 2, welche eine Größe von 1-3 µm haben.

4. Teilchen gemäß mindestens einem der vorhergehenden Ansprüche, wobei das lebende Tier ein Mensch ist.

## Revendications

1. Particules support destinées à être utilisées dans une méthode de thérapie par transformation génétique *in vivo* de cellules somatiques dans la peau d'un animal vivant, dans lesquelles les particules sont en matériau dense, sont petites par rapport à la taille desdites cellules, et sont revêtues avec des copies d'une construction génétique exogène qui comprend une séquence d'ADN codant une protéine et des séquences régulatrices adjacentes efficaces pour exprimer la protéine dans lesdites cellules.

2. Particules selon la revendication 1 qui sont en or.

3. Particules selon la revendication 2, qui ont une taille de 1-3 µm.

4. Particules selon l'une quelconque des revendications précédentes, dans lesquelles l'animal vivant est un être humain.
